# FASCICULE DE BREVET EUROPEEN

(11) **EP 1 242 127 B1**
(45) Date de publication et mention de la délivrance du brevet: **27.08.2003**
(21) Numéro de dépôt: 00993690.7
(22) Date de dépôt: 26.12.2000
(51) Int. Cl.: A61L 15/46, A61L 15/50

(54) **TAMPON PERIODIQUE ANTISEPTIQUE ET SON PROCEDE DE PREPARATION**
ANTISEPTISCHER VAGINALTAMPON UND VERFAHREN ZU DESSEN HERSTELLUNG
ANTISEPTIC TAMPON AND PREPARATION METHOD

(30) Priorité: 29.12.1999 FR 9916847
(43) Date de publication de la demande: 25.09.2002
(73) Titulaire: Pasquini, Jean-Bastien, 3335 Leudelange (LU)
(72) Inventeur: PASQUINI, Jean-Bastien, F-3335 Leudelange (LU); PASQUINI, Michel, F-24200 Carsac-Aillac (FR)
(74) Mandataire: Cabinet Martinet & Lapoux
(86) Numéro de dépôt international: FR0003681
(87) Numéro de publication internationale: WO01049332

(56) Documents cités:
- WO-A-00/37118
- DE-A- 2 309 575
- US-A- 3 756 238
- US-A- 5 417 224

## Description

L'invention concerné un tampon périodique antiseptique et un procédé de préparation d'un tel tampon.

On sait qu'un tampon périodique d'un type standard constitue un foyer d'infection potentielle du milieu vaginal, parce que sa mise en place peut s'accompagner d'une introduction dans ce milieu de germes plus ou moins pathogènes, présents au niveau de la vulve et de la surface cutanée avoisinante. En outre, ce tampon en matière absorbante a un effet desséchant sur les muqueuses vaginales, qui les fragilise et les rend plus sensibles à diverses infections.

Pour réduire cet inconvénient, on a proposé de réaliser des tampons en matière moins absorbante, qu'il faut changer plus fréquemment, par exemple toutes les quatre heures, ce qui est très peu commode et très contraignant.

On a également proposé, notamment dans le document EP-A-0 110 793, un tampon périodique dont une extrémité comporte un alvéole en forme de cupule et un ovule contenant une matière antiseptique ou médicamenteuse, fixé dans l'alvéole par une gomme adhésive soluble. Lorsque ce tampon est mis en place, l'ovule se trouve au voisinage du col de l'utérus et permet d'aseptiser le milieu vaginal et le flux de sang qui s'écoule dans le vagin.

Ce tampon se prête toutefois assez mal à une fabrication économique en grande série, notamment en raison de la formation d'un alvéole à l'extrémité du tampon, de l'amenée d'une gomme adhésive soluble et de la pose d'un ovule de matière antiseptique ou médicamenteuse dans l'alvéole.

Le document US-A-3 756 238 déjà décrit un tampon dont d'une partie est recouverte d'une couche lubrifiante contenant des bactéricides, des fongicides, des antibiotiques etc. La couche est composée d'un polymère thermoplastique, hydrosoluble, d'un plastifiant organique et d'un lubrifiant; ainsi la couche forme un revêtement hydrophile.

Le document WO 0037118 décrit l'utilisation d'un mélange de produit antiseptique et de produit hydrophobe pour imprégner un tampon absorbant.

La présente invention a notamment pour but d'apporter une solution simple, efficace et économique aux problèmes cités ci-dessus.

Elle a pour objet un tampon périodique antiseptique, qui évite les risques d'infection du milieu vaginal, sans fragilisation des muqueuses vaginales.

Elle a également pour objet un tampon périodique aseptique, qui est préparé à partir d'un tampon périodique d'un type standard.

Elle a encore pour objet un procédé de préparation d'un tampon périodique antiseptique qui est économique et qui se prête à une fabrication automatique en grande série sur des machines d'un type connu.

L'invention propose à cet effet un procédé de préparation d'un tampon périodique réalisé en matière absorbante et comprenant un produit antiseptique, caractérisé en ce qu'il consiste à introduire des quantités dosées d'un mélange liquide de produit antiseptique et d'excipient hydrophobe dans des gaines de conditionnement individuel de tampon, puis à introduire les tampons dans lesdites gaines de sorte que leurs extrémités destinées à être revêtues dudit mélange se trouvent au fond des gaines en contact avec le mélange, et à fermer de façon étanche lesdites gaines.

La préparation des tampons périodiques selon l'invention s'effectue ainsi de façon concomitante avec leur conditionnement, d'une façon particulièrement simple et économique.

Selon encore une autre caractéristique de l'invention, ce procédé consiste également à préparer le mélange de produit antiseptique et d'excipient hydrophobe par fusion à chaud de l'excipient hydrophobe, ajout du produit antiseptique dans l'excipient fondu et mélange du produit antiseptique et de l'excipient fondu avant coulée dans les gaines de conditionnement des tampons, puis après fermeture étanche des gaines, à solidifier le mélange de produit antiseptique et d'excipient hydrophobe sur les tampons par refroidissement.

On obtient ainsi un revêtement solide, antiseptique, hydrophobe et lubrifiant de la partie d'extrémité du tampon qui sert à son introduction dans le vagin.

Avantageusement, le procédé selon l'invention consiste également à thermoformer des alvéoles semi-cylindriques dans deux bandes de matière plastique par exemple en PVC, ces alvéoles débouchant sur un bord latéral desdites bandes, à appliquer les deux bandes l'une sur l'autre face à face pour former lesdites gaines par réunion de deux alvéoles semi-cylindriques, et à fixer les deux bandes l'une sur l'autre par thermoscellage ou thermosoudure.

Un tel procédé est exécutable sur des machines d'un type connu, utilisées notamment pour la fabrication de suppositoires, et nécessite donc un investissement faible en matériel, puisqu'il suffit d'adapter des machines existantes.

Selon une autre caractéristique de l'invention, on ajoute, au mélange d'excipient et de produit antiseptique avant coulée dans les gaines de conditionnement des tampons, du bacille de Döderlein (lactobacillus casei), par exemple sous forme lyophilisée et à un dosage de l'ordre de 90 mg par tampon.

Les tampons ainsi préparés sont destinés à être utilisés à la fin des règles, pour ré-ensemencer la flore normalement présente dans le vagin.

L'invention propose également un tampon périodique antiseptique, préparé par exécution du procédé décrit ci-dessus, caractérisé en ce que le mélange du produit antiseptique et de l'excipient hydrophobe forme un revêtement d'une partie d'extrémité du tampon.

Ce mélange de produit antiseptique et d'excipient hydrophobe revêt la partie d'extrémité du tampon se trouvant au voisinage du col de l'utérus lorsque le tampon est en place et évite tout risque de dessèchement et de fragilisation des muqueuses vaginales avec de plus un effet antiseptique sur le milieu vaginal qui est protégé des risques d'infection.

Avantageusement, l'excipient hydrophobe est un lubrifiant, qui facilite la mise en place du tampon et évite l'utilisation d'un applicateur.

Dans un mode de réalisation préféré de l'invention, le produit antiseptique est du nonoxynol 9.

Ce produit est utilisé depuis plus de trente ans comme spermicide dans de nombreuses préparations pour applications vaginales et sa bonne tolérance locale et générale a été constatée par de multiples études.

Selon une autre caractéristique de l'invention, ce tampon périodique antiseptique est préparé à partir d'un tampon périodique standard.

Cette caractéristique réduit assez fortement le prix de revient du tampon selon l'invention.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 représente schématiquement les étapes essentielles du procédé selon l'invention ;
- la figure 2 est une vue schématique d'un tampon selon l'invention dans son conditionnement individuel.

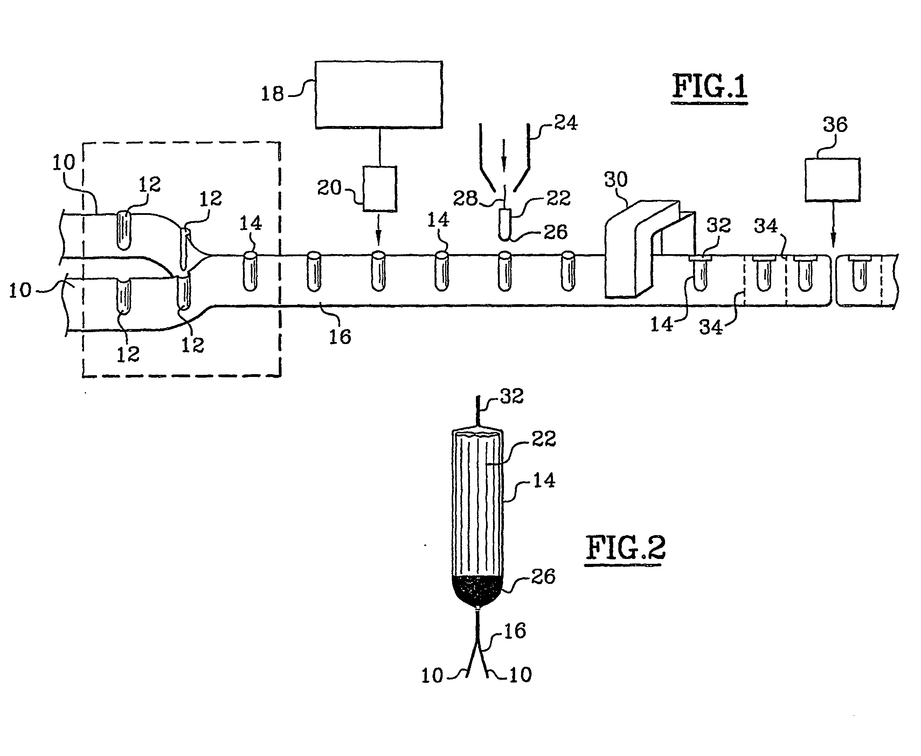

Dans le procédé représenté en figure 1, la première étape consiste à dérouler deux bandes 10 d'une matière plastique thermoscellable, telle par exemple que du poly(chlorure de vinyle) et à former dans chacune de ces bandes des alvéoles semi-cylindriques 12 par thermoformage, ces alvéoles débouchant par une extrémité sur un bord latéral de la bande 10 correspondante, tandis que leur autre extrémité est à distance de l'autre bord latéral de la bande 10.

Les deux bandes 10 sont ensuite rapprochées l'une de l'autre et appliquées l'une sur l'autre, leurs alvéoles semi-cylindriques 12 étant sensiblement verticaux et en regard les uns des autres pour former des gaines cylindriques 14 ouvertes vers le haut.

Les deux bandes 10 sont fixées l'une à l'autre pour former une bande unique, à l'exception d'une zone latérale 16 où les deux bandes 10 restent séparées l'une de l'autre, cette zone latérale 16 s'étendant le long du bord latéral des bandes 10 situé du côté du fond des gaines 14.

Dans un mode de réalisation préféré de l'invention, les deux bandes 10 sont fixées par thermosoudure, à une température d'environ 200°C.

L'étape suivante du procédé consiste à verser dans chaque gaine 14 une quantité dosée d'un mélange liquide contenant un produit antiseptique et un excipient hydrophobe et lubrifiant.

Ce mélange est préparé par fusion de l'excipient, qui est avantageusement à base de glycérides, tel que celui commercialisé sous la dénomination SUPPOCIRE AM par GATTEFOSSE. Ce produit est placé dans une cuve 18 à parois chauffantes thermorégulées. On peut par exemple chauffer cette cuve à une température de 50-55°C environ, puis y placer une certaine quantité d'excipient, par exemple d'environ 60 kg, et maintenir ensuite l'excipient fondu dans la cuve à une température de l'ordre de 38°C. La cuve 18 est équipée d'un agitateur à pales, permettant de mélanger l'excipient dans la cuve jusqu'à fusion totale.

On ajoute ensuite dans la cuve une quantité prédéterminée d'un produit antiseptique tel que de préférence le nonoxynol 9, qui est un liquide visqueux anhydre, principalement composé d'éthers mononylphényliques de polyéthènes glycols, de formule générale C9H19C6H4-(OCH2-CH2)n-OH où n est en général égal à 9 mais peut être compris entre 6 et 16.

On introduit par exemple dans la cuve 18, 5 kg de nonoxynol 9 pour 65 kg d'excipient et on mélange pendant environ 30 minutes à 38°C pour obtenir un mélange homogène.

La cuve 18 est reliée à un appareil de dosage 20 d'un type connu, permettant de couler le mélange de produit antiseptique et d'excipient dans les gaines 14, à raison par exemple de 700 milligrammes par gaine, pour remplir les extrémités inférieures des gaines.

Ensuite, des tampons périodiques 22 d'un type standard sont introduits dans les gaines 14, par exemple à la main ou au moyen d'un système 24 d'alimentation automatique, de façon à ce que l'extrémité arrondie 26 du tampon plonge dans le mélange contenu dans le fond de la gaine 14.

Le tampon périodique 22 d'un type standard est fabriqué à partir de fibres naturelles (viscose et/ou coton) contenues dans un voile doux en matériau textile non tissé, et comporte éventuellement un cordon 28 d'extraction en matière hydrophobe.

Les gaines 14 contenant les tampons 22 défilent ensuite dans un poste 30 de scellement et de marquage où l'extrémité supérieure 32 de chaque gaine 14 est scellée de façon étanche et reçoit par impression un numéro de lot et une date de péremption.

Les gaines 14 passent ensuite dans un tunnel de refroidissement à température ambiante où le mélange de produit antiseptique et d'excipient revêtant la partie d'extrémité inférieure de chaque tampon 22 est solidifié.

Des lignes transversales de prédécoupe 24 sont formées dans les bandes 10, entre les gaines 14.

Des moyens de coupe 36 sont ensuite prévus pour découper les bandes 10 en plaquettes comprenant des nombres prédéterminés de tampons périodiques antiseptiques conditionnés dans les gaines 14. Ces plaquettes sont elles-mêmes conditionnables dans des étuis en carton sur lesquels les numéros de lot et les dates de péremption correspondantes sont imprimées.

La figure 2 représente schématiquement, à plus grande échelle, un tampon périodique selon l'invention dans sa gaine 14 de conditionnement individuel.

L'extrémité inférieure 26 du tampon 22 comporte un revêtement solide du mélange de produit antiseptique et d'excipient hydrophobe et lubrifiant, et l'extrémité inférieure de la gaine 14 est prolongée par deux volets indépendants formés par les parties des bandes 10 comprises dans la zone libre 16 entre deux prédécoupes transversales 34, et qui permettent d'ouvrir facilement la gaine par traction sur ces deux volets.

La quantité de produit antiseptique que comporte chaque tampon périodique peut bien entendu varier dans une certaine mesure. Elle est inférieure à 150 milligrammes environ et de préférence de l'ordre de 50 milligrammes dans le cas du nonoxynol 9. De même, la quantité d'excipient hydrophobe et lubrifiant peut varier autour de la valeur de 650 milligrammes par tampon, par exemple entre 500 et 800 milligrammes environ.

La préparation des tampons périodiques antiseptiques selon l'invention, qui est faite en utilisant des tampons périodiques standards et des machines existantes de fabrication de suppositoires, est très économique et nécessite un investissement relativement faible.

Le produit antiseptique et l'excipient hydrophobe et lubrifiant utilisés sont très bien tolérés et ne présentent à peu près aucune contre-indication, de sorte que le tampon périodique antiseptique selon l'invention ne provoque aucune irritation des muqueuses vaginales et, après tests, a été classé parmi les matériaux non sensibilisants par contact avec la peau.

Un usage répété de ce tampon permet de réduire et de prévenir les risques d'infection et renforce la protection des muqueuses vaginales.

Le tampon selon l'invention peut également être utilisé pour ré-ensemencer la flore normalement présente dans le vagin, à la fin des règles. Pour cela, on ajoute du bacille de Döderlein (bacillus casei) au mélange de produit antiseptique et d'excipient coulé dans les gaines de conditionnement 14. Le bacille de Döderlein est disponible sous forme lyophilisée et est dosé à une quantité de l'ordre de 80 à 120 mg par tampon, de préférence d'environ 90 mg.

## Revendications

1. Procédé de préparation d'un tampon périodique réalisé en matière absorbante et comprenant un produit antiseptique, **caractérisé en ce qu'**il consiste :
- à introduire des quantités dosées d'un mélange liquide de produit antiseptique et d'excipient hydrophobe dans des gaines (14) de conditionnement individuel de tampons périodiques (22),
- puis à introduire des tampons périodiques (22) dans lesdites gaines (14) de sorte que leurs extrémités destinées à être revêtues dudit mélange se trouvent au fond desdites gaines en contact avec le mélange,
- et à fermer de façon étanche lesdites gaines (14).

2. Procédé selon la revendication 1, **caractérisé en ce qu'**il consiste à préparer le mélange de produit antiseptique et d'excipient par fusion à chaud de l'excipient, addition du produit antiseptique dans l'excipient fondu et mélange du produit antiseptique et de l'excipient avant coulée du mélange dans les gaines (14) de conditionnement, puis après fermeture des gaines (14), à solidifier le mélange sur les tampons (22) par refroidissement.

3. Procédé selon la revendication 1 ou 2, **caractérisé en ce qu'**il consiste à ajouter du bacille de Döderlein dans le mélange de produit antiseptique et d'excipient, par exemple dans une quantité de l'ordre de 90 mg par tampon, avant coulée du mélange dans les gaines (14).

4. Procédé selon l'une des revendications 1 à 3, **caractérisé en ce qu'**il consiste à thermoformer des alvéoles semi-cylindriques (12) dans deux bandes de matière plastique telles par exemple que du PVC, les alvéoles (12) débouchant sur un bord latéral desdites bandes, à appliquer les deux bandes face à face pour former lesdites gaines (14) par réunion de deux alvéoles (12) en regard l'un de l'autre, et à fixer les deux bandes (10) l'une à l'autre par thermosoudure.

5. Tampon périodique antiseptique préparé par exécution du procédé selon l'une des revendications 1 à 4, **caractérisé en ce que** le mélange du produit antiseptique et de l'excipient hydrophobe forme un revêtement d'une partie d'extrémité (26) du tampon.

6. Tampon selon la revendication 5, **caractérisé en ce que** le produit antiseptique est du nonoxynol 9.

7. Tampon selon la revendication 5 ou 6, **caractérisé en ce que** l'excipient hydrophobe est lubrifiant et est en particulier à base de glycérides.

8. Tampon selon l'une des revendications 5 à 7, **caractérisé en ce que** le mélange comprend une quantité de produit antiseptique inférieure ou égale à 150 milligrammes environ, et de préférence de l'ordre de 50 milligrammes.

9. Tampon selon l'une des revendications 5 à 8, **caractérisé en ce que** ledit mélange comprend de 500 à 800 milligrammes environ d'excipient hydrophobe.

10. Tampon selon l'une des revendications 5 à 9, **caractérisé en ce qu'**il est conditionné individuellement dans une gaine scellée (14) en matière plastique, telle par exemple que du poly(chlorure de vinyle).

11. Tampon selon l'une des revendications 5 à 10, **caractérisé en ce qu'**il est préparé à partir d'un tampon périodique standard.

12. Tampon selon l'une des revendications 5 à 11, **caractérisé en ce que** le revêtement de son extrémité (26) contient du bacille de Döderlein, et **en ce qu'**il est destiné à être utilisé en fin de règles.

## Patentansprüche

1. Verfahren zur Herstellung eines Vaginaltampons, ausgeführt mit absorbierendem Material und umfassend ein antiseptisches Erzeugnis, **dadurch gekennzeichnet, daß** es darin besteht,
- dosierte Mengen einer flüssigen Mischung eines antiseptischen Erzeugnisses und einer hydrophoben Masse in Einzelverpackungsumhüllungen (14) für Vaginaltampons (22) einzuführen,
- dann Vaginaltampons (22) in die Umhüllungen (14) einzuführen derart, daß ihre für einen Überzug mit der Mischung bestimmten Enden sich am Boden der Umhüllungen in Kontakt mit der Mischung befinden,
- und die Umhüllungen (14) dicht zu verschließen.

2. Verfahren nach Anspruch 1, **dadurch gekennzeichnet,daß** es darin besteht, die Mischung aus dem antiseptischen Erzeugnis und der Masse durch Heißschmelzen der Masse, Zusatz des antiseptischen Erzeugnisses in die geschmolzene Masse und Mischung des antiseptischen Erzeugnisses und der Masse vor Gießen der Mischung in die Verpackungsumhüllungen (14) herzustellen, dann nach Verschließen der Umhüllungen (14) die Masse auf den Tampons (22) durch Kühlung zu verfestigen.

3. Verfahren nach Anspruch 1 oder 2, **dadurch gekennzeichnet,daß** es darin besteht, Döderlein Stäbchen in die Mischung aus antiseptischem Erzeugnis und Masse, beispielsweise mit einer Menge in der Größenordnung von 90 mg pro Tampon vor Gießen der Mischung in die Umhüllungen (14) zuzufügen.

4. Verfahren nach einem der Ansprüche 1 bis 3, **dadurch gekennzeichnet, daß** es darin besteht, halbzylindrische Waben (12) in zwei Streifen aus Kunststoffmaterial wie beispielsweise aus PVC wärmezuformen, wobei die Waben (12) in eine Seitenkante der Bänder münden, die beiden Bänder aufeinander aufzubringen, um die Umhüllungen (14) durch Vereinigung von zwei Waben (12) einander gegenüber zu bilden, und die beiden Bänder (10) durch Heißsiegeln zu befestigen.

5. Antiseptischer Vaginaltampon, hergestellt durch Ausführung des Verfahrens nach einem der Ansprüche 1 bis 4, **dadurch gekennzeichnet, daß** die Mischung aus dem antiseptischen Erzeugnis und der hydrophoben Masse einen Überzug eines Endteils (26) des Tampons bildet.

6. Tampon nach Anspruch 5, **dadurch gekennzeichnet,daß** das antiseptische Erzeugnis Nonoxinol 9 ist.

7. Tampon nach Anspruch 5 oder 6, **dadurch gekennzeichnet,daß** das hydrophobe Erzeugnis Schmiermittel ist und insbesondere auf Basis von Glyceriden ist.

8. Tampon nach einem der Ansprüche 5 bis 7, **dadurch gekennzeichnet,daß** die Mischung eine Menge an antiseptischem Erzeugnis kleiner oder gleich ungefähr 150 Milligramm und vorzugsweise in der Größenordnung von 50 Milligramm umfaßt.

9. Tampon nach einem der Ansprüche 5 bis 8, **dadurch gekennzeichnet,daß** die Mischung ungefähr 500 bis 800 Milligramm an hydrophobem Erzeugnis enthält.

10. Tampon nach einem der Ansprüche 5 bis 9, **dadurch gekennzeichnet,daß** er einzeln in einer versiegelten Umhüllung (14) aus Kunststoff wie beispielsweise aus Poly (Vinylchlorid) verpackt ist.

11. Tampon nach einem der Ansprüche 5 bis 10, **dadurch gekennzeichnet, daß** er ausgehend von einem Standard-Vaginaltampon hergestellt worden ist.

12. Tampon nach einem der Ansprüche 4 bis 11, **dadurch gekennzeichnet, daß** der Überzug seines Endes (26) Döderlein Stäbchen enthält und daß er zur Verwendung am Periodenende bestimmt ist.

## Claims

1. A method of preparing a tampon made from an absorbent material and containing an antiseptic product, **characterized in that** it consists of:
- introducing metered quantities of a liquid mixture of antiseptic product and hydrophobic excipient into individual packaging sheaths (14) of tampons (22),
- then introducing tampons (22) into said sheaths (14) so that their ends intended to be covered with said mixture are at the bottom of said sheaths and in contact with the mixture, and
- closing and sealing said sheaths (14).

2. A method according to claim 1, **characterized in that** it consists of preparing the mixture of antiseptic product and excipient by heating the excipient to melt it, adding the antiseptic product to the molten excipient and mixing the antiseptic product and the excipient before pouring the mixture into the packaging sheaths (14), and then after closing the sheaths (14), solidifying the mixture on the tampons (22) by cooling.

3. A method according to claim 1 or 2, **characterized in that** it consists of adding Döderlein's bacillus to the mixture of an antiseptic product and excipient, for example at a rate of the order of 90 mg per tampon, before pouring the mixture into the sheaths (14).

4. A method according to any one of claims 1 to 3, **characterized in that** it consists of thermoforming semicylindrical cells (12) in two strips of plastics material, such as PVC, for example, the cells (12) opening onto a lateral edge of said strips, applying the two strips face-to-face to form said sheaths (14) by joining two facing cells (12), and heat-welding the two strips (10) together.

5. An antiseptic tampon prepared by executing the method according to any one of claims 1 to 4, **characterized in that** the mixture of the antiseptic product and the hydrophobic excipient forms a covering of an end portion (26) of the tampon.

6. A tampon according to claim 5, **characterized in that** the antiseptic product is nonoxynol 9.

7. A tampon according to claim 5 or 6, **characterized in that** the hydrophobic excipient is a lubricant and is in particular based on glycerides.

8. A tampon according to any one of claims 5 to 7, **characterized in that** the mixture contains a quantity of antiseptic product less than or equal to approximately 150 mg and preferably of the order of 50 mg.

9. A tampon according to any one of claims 5 to 8, **characterized in that** said mixture contains approximately 500 to 800 mg of hydrophobic excipient.

10. A tampon according to any one of claims 5 to 9, **characterized in that** it is individually packaged in a sealed sheath (14) of plastics material, for example polyvinyl chloride.

11. A tampon according to any one of claims 5 to 10, **characterized in that** it is prepared from a standard tampon.

12. A tampon according to any one of claims 5 to 11, **characterized in that** the covering of its end (26) contains Döderlein's bacillus and **in that** it is intended to be used at the end of a menstrual period.
